# EUROPEAN PATENT APPLICATION

(11) **EP 1 609 461 A1**
(43) Date of publication of application: **28.12.2005**
(21) Application number: 05013322.2
(22) Date of filing: 21.06.2005
(51) Int. Cl.: A61K 7/48, A61K 7/06

(54) **Cosmetic compositions comprising a mixture of N-acyl-phosphatidylethanolamines and cosmetic treatments thereof.**

(30) Priority: 24.06.2004 IT mi20041280
(71) Applicant: Hunza di Pistolesi Elvira & C. S.a.S., 20148 Milan (IT)
(72) Inventor: Pistolesi, Elvira, 20148 Milano (IT); Cestaro, Benvenuto, 20148 Milano (IT)
(74) Representative: Minoja, Fabrizio

(57) **Abstract**

The present invention relates to cosmetic compositions of the topical use consisting of mixtures of N-acyl-phosphatidylethanolamines and optionally bioflavones.

## Description

### SUMMARY OF THE INVENTION

The present invention relates to cosmetic compositions of the topical use consisting of mixtures of N-acyl-phosphatidylethanolamines and optionally bioflavones.

### TECHNOLOGICAL BACKGROUND

N-acyl-phosphatidylethanolamines (NAFEs) are compounds naturally present in a number of animal and vegetable sources (H.H. Schmid et al., 1990, *Prog. Lipid Res*., 29, 1-43) and are particularly abundant in soy, eggs, meat and chocolate (K.D. Chapman et al., 1993, *Arch. Biochem. Biophys.,* 301, 21-23; E. Di Tomaso et al., 1996, *Nature*, 382, 677-678).

GB 2551069 claims the anti-lipemic and anti-atherosclerotic effect of N-oleyl-phosphatidylethanolamine (NOFE) administered per os, but no mention is made of any other activities of either NOFE or other NAFEs.

WO 03/068210-A1 claims the antioxidant, membrane-fluidifying, mitochondrion energetic activity improving and body weight controlling actions of mixtures of NAFEs (with the exclusion of N-oleyl-phosphatidylethanolamine) administered per os either as such or in the form of complexes with one or more bioflavones.

EP 0606590 claims the dermatological or cosmetic use of co-mixtures comprising both NAFEs and N-acyl-ethanolamine (NAE) for the treatment of the skin damages induced by heat, sun radiation or insect bites.

### DISCLOSURE OF THE INVENTION

The present invention relates to topical cosmetic compositions consisting of NAFEs and optionally comprising bioflavones, useful for improving the quality, growth and aspect of the hair and for reducing both the unaesthetic appearance of cellulite and the wrinkle and unaesthetic appearance typical of the aging skin.

The compositions of the invention consist therefore of NAFE (Component A as defined below) and optionally comprising bioflavones (Component B) and/or suitable buffers ( Component C), wherein:
A) represents N-acyl-phosphatidylethanolamines (NAFEs) alone or in phospholipid mixtures (containing phosphatidylcholine and/or phosphatidylethanolamine and/or phosphatidylserine and/or phosphatidylinositol and/or phosphatidylglycerol and/or the corresponding lysoderivatives thereof); NAFE alone or in mixture with the other phospholipids being present either as such or in the form of salts (e.g. Ca, Mg, Zn, Na, K, Mn, Se, Cr, Va, etc.) compatible with the cosmetic use in humans.
   The structural formula of NAFEs is reported hereinbelow: 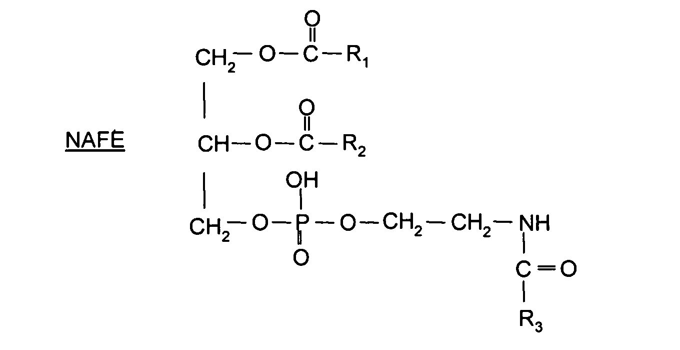 wherein R1, R2 and R3 are C12-C20 alkyl chains of saturated, mono- or polyunsaturated fatty acids, such as palmitic, stearic, oleic, linoleic, linolenic, conjugated linoleic (CLA) and eicosapentaenoic acids;
B) represents one or more bioflavones selected from polyphenols, flavones, flavonols, isoflavones, proanthocyanidins, tannins, etc.;
C) one or more dehydrated buffering agents able to stabilize the final pH from 3.5 to 6.0.

According to a preferred aspect of the invention, NAFEs wherein R3 fatty acids consist of acid oleic (NOFE) are used, as they proved the most active in promoting the desired cosmetic effects.

Preferred compositions of the invention include both component A and component B and optionally the buffering agents C.

According to a preferred aspect of the invention, epigallocatechin gallate (from green tea) is used, as it proved the most active in promoting the cosmetic effects according to the present invention.

According to a preferred aspect of the invention, the concentration of bioflavones ranges from 0.5 to 20%, preferably from 5 to 10% of the total phospholipids present.

Said mixtures in powder forms of NAFEs and bioflavones can be used as such for:
1) Preparing aqueous emulsions containing the active principles aggregated as water-dispersed phosphobioflavone complexes which spontaneously form in water upon stirring and are characterized by an "hexagonal phase" structure (NAFE > 80% of the total phospholipids present) and/or "liposomal" (NAFE < 80% of the total of the phospholipids present) and/or "micellar" (phospholipid lysoderivatives > 60% of the total phospholipids present).
   The invention also relates to said water-dispersed phosphobioflavone complexes in the hexagonal and/or liposomal and/or micellar phase.
2) Preparing oily emulsions containing the active principles aggregated as oily dispersed phosphobioflavone complexes characterized by "retro-micellar" structure. Said oily dispersed phosphobioflavone complexes with retro-micellar structure are a further object of the present invention. The retro-micellar structures containing the active principles (phospholipid mixtures of NAFE and bioflavones) form spontaneamente when the co-mixed powders of the active principles are dissolved and stirred in the suitable oily solutions.

The oily solutions used are preferably vegetable and/or animal and/or mineral oils of various nature (e.g. maize, olive, soy, bitter almond, avocado, jojoba, wheat germ, sesame, hazelnut, palm, flaxseed, vaseline, medium chain triglycerides etc.).

The water-dispersed phosphobioflavone complexes with hexagonal and/or liposomal and/or micellar phase (see point 1° above) and the oily dispersed phosphobioflavone complexes with retro-micellar structure (see point 2° above) of co-phospholipid mixtures containing NAFEs and one or more bioflavones proved surprisingly effective also in increasing the cutaneous absorption and the bioavailability of the bioflavones.

The cosmetic effects claimed by the present invention can be further improved by adding the NAFEs and bioflavones co-mixed powders with one or more of the following classes of compounds:
a) fibres of various origin (cellulose, pectins, inulins, chitosans, etc.).
b) mixtures of peptides, amino acids and derivatives thereof (creatin, S-adenosylmethionine, carnitine, carnosine, glutathione, aspartic acid, cysteine, arginine, glutamine, etc.).
c) extracts of plants and/or fruits (containing essential oils rich in mono- and di-terpenes, saponins, carotenes, etc.).
vitamins (A, group B, C, D, AED, K, H, etc.) and vitamin-like factors (lipoic acid, CoQ, tocotrienols, etc.).

According to a preferred aspect of the invention, particularly preferred are water soluble oligomeric fibres of glucosamine obtained by hydrolysis and de-acetylation of chitin polymeric chains.

According to a preferred aspect of the invention, particularly preferred are mixtures consisting of arginine and glutathione.

According to a preferred aspect of the invention, particularly preferred is a mixture consisting of one or more monoterpenes selected form d-limonene, eugenol, thymol, and diterpenes suc as phytol.

According to a preferred aspect of the invention, particularly preferred is a mixture consisting of one or more of the compounds selcted from pantotenic acid, nicotinamide, biotin, tocopherols and derivatives, ascorbic acid and derivatives.

The compositions according to the present invention can be in any cosmetic form normally used for the topical application to the skin; particularly preferred are the forms of the water-dispersed phosphobioflavonic complexes and oily-dispersed phosphobioflavonic complexes.

The present compositions may be more or less fluid and have the appearance of a white or coloured cream, lotion, oinment, milk, serum, paste, mousse or gel. They can optionally be applied to the skin and/or hair in the form of an aerosol. They can also be in solid form, for example, in the form of a stick. They can be used as a care product and/or as a make-up product for the skin and/or hair.

In a known manner, the composition of the present invention can also contain adjuvants conventionally used in cosmetics and dermatology, such as hydrophilic or lipophilic gelling agents, emulsifiers and co-emulsifiers, hydrophilic or lipophilic active agents, preservatives, antioxidants, solvents, fragrances, fillers, pigments, hydrophilic screening agents, odor absorbers and colorants. The amounts of these adjuvants are those conventionally used in the fields under consideration, for example, from 0.01 to 20% by weight of the total weight of the composition. Depending on their nature, these adjuvants can be introduced into the fatty phase, into the aqueous phase and/or into the lipid vesicles (liposomal, micellar and hexagonal phase).

As noted above, the present compositions may be used for improving the quality and aspect of hair and preventing its loss or for combating the unaesthetic appearances of cellulite and/or aging skin of a subject in need thereof. When using the present compositions in such a manner, the compositions are suitably applied to the skin and/or hair in an amount of 0.1 to 500 mg/cm², preferably 10 to 100 mg/cm².

The compositions may be applied to the skin and/or to the hair daily or twice daily or more. The application of the compositions may be continued until the desired degree of improvement is achieved or continued indefinitely for preventative purposes.

The amounts of the different active compounds in 100 g of the compositions (lotion, cream, milk, etc.) are the following:
- NAFEs, particularly NOFE: 0.01 to 5%, preferably 0.2. to 0.5%.
- bioflavones (and in particular epigallocathechingallate): 0.001 to 0.5%, preferably 0.02 to 0.05%.
- glucosamine hydrosoluble oligomers: 0.02 to 10%, preferably 0.4 to 1%.
- arginine: 0.05 to 5%, preferably 0.5 to 1.5%.
- glutathione: 0.01 to 1%, preferably 0.075 to 0.125%.
- mixtures of mono-and di- terpenes, and in particular one or more of the following compounds: d-limonene, eugenol, timol and phytol: 0.001 to 2.5%, preferably 0.05 to 0.5%.
- pantotenic acid: 0.001 to 5%, preferably 0.1 to 0.7%.
- niacinamide: 0.01 to 1%, preferably 0.075 to 0.125%.
- biotine: 0.001 to 0.2%, preferably 0.005 to 0.02%.
- tocoferols: 0.001 to 0.5%, preferably 0.01 to 0.04%.
- ascorbic acid: 0.01 to 2%, preferably 0.1 to 0.4%.

The invention is illustrated in greater detail by the following examples.

### EXAMPLE 1: co-mixed powders of phospholipids

The following powder ingredients are mixed by mechanical stirring:
- 250 mg of NOFE
- 2.25 g of soy lecithins (phosphatidylcholine, phosphatidylethanolamine and other minor phospholipids.

### EXAMPLE 2: co-mixed powders of phospholipids and bioflavones

The following powder ingredients are mixed by mechanical stirring:
- 250 mg of NOFE
- 2.25 g of soy lecithins (phosphatidylcholine, phosphatidylethanolamine and other minor phospholipids
- 100 mg of green tea epigallocatechin

### EXAMPLE 3: co-mixed powders of phospholipids and bioflavones

The following powder ingredients are mixed by mechanical stirring:
- 250 mg of NOFE
- 1.75 g of soy lysolecithins (lysophosphatidylcholine and lysophosphatidylethanolamine more other minor lysophospholipids)
- 100 mg of green tea epigallocatechin

### EXAMPLE 4: Co-mixed powders of phospholipids, bioflavones, vitamins, amino acids and peptides and glucosamine oligomers

The following powder ingredients are mixed by mechanical stirring:
- 250 mg of NOFE
- 2.25 g of soy lecithin
- 100 mg of green tea epigallocatechin
- 500 mg of chitin de-acetylated oligomers
- 1 g of arginine
- 100 mg of glutathione
- 250 mg of pantotenic acid
- 100 mg of niacinamide
- 20 mg of biotin
- 20 mg of tocopherols
- 300 mg of ascorbyl phosphate

### EXAMPLE 5: Co-mixed powders of phospholipids and bioflavones

The following powder ingredients are mixed by mechanical stirring:
- 50 g of NOFE
- 10 g of green tea epigallocatechin

### EXAMPLE 6

2.6 g of the preparation of co-mixed powders of phospholipids and bioflavones of Example 2 are dissolved in 97.4 g of an aqueous solution buffered to pH 5.5 and mechanically stirred for some minutes. An aqueous emulsion spontaneously forms containing the active principles aggregated in water-dispersible phosphobioflavone complexes characterized by the liposomal structure of the invention.

### EXAMPLE 7

2.1 g of the preparation of co-mixed powders of phospholipids and bioflavones of Example 3 are dissolved in 97.9 g of an aqueous solution buffered to pH 5.5 and mechanically stirred for some minutes. An aqueous emulsion spontaneously forms containing the active principles aggregated in water-dispersible phosphobioflavone complexes characterized by the micellar structure of the invention.

### EXAMPLE 8

6 g of the preparation of co-mixed powders of phospholipids and bioflavones of Example 5 are dissolved in 994 g of an aqueous solution buffered to pH 5.5 and sonicated for some minutes. An aqueous emulsion spontaneously forms containing the active principles aggregated in water-dispersible phosphobioflavone complexes characterized by the hexagonal phase structure of the invention.

### EXAMPLE 9

2.6 g of the preparation of co-mixed powders of phospholipids and bioflavones of Example 2 are dissolved in 97.4 g of a oily solution (consisting of 60% oil of maize and 40% medium chain triglycerides) and mechanically stirred for some minutes. An oily emulsion spontaneously forms containing the active principles aggregated in oily dispersed phosphobioflavone complexes characterized by the retro-micellar structure of the invention.

### COSMETIC TESTS

The compositions of the invention have demonstrated a significant cosmetic activity since they improve or enhance the general appearance of the hair and/or skin (in this second case preventing and/or ameliorating the unaesthetic appearances of cellulite and/or skin-ageing) of the individual user.

### HAIR TREATMENT

To this end, a few notions regarding the phases of growth of hair should be presented. The growth cycle of hair consists of three phases: I) the anagen phase that corresponds to the phase of hair growth during which the dermal papilla undergoes intense metabolic activity; II) the catagen phase during which the hair follicle undergoes morphological and metabolic changes (the length of the follicle is reduced, the bulb decreases in size, the melanocytes stop producing pigment) and, finally the III) telogenic phase or resting phase, during which the hair follicle is completely inactive.

Every day about 50 hairs die and fall out, and, under normal conditions, are replaced by new members so that the total volume of hair remains virtually unchanged.

The factors that can lead to an imbalance in these hair cycles may be different: non-balanced diet, prolonged states of stress, anxiety, use of drugs (e.g.: bromocryptine, cimetidine, lithium, etc.), deficiencies in vitamins and minerals, alcohol abuse, etc.).

This invention features a cosmetic hair treatment for improving the appearance of the hair and/or for promoting regrowth of the hair and/or for decreasing hair loss, comprising daily topically applying at least one of the compositions described above onto the scalp and/or the hair for a period of at least 30 days or more.

The compositions of the invention have been found to improve the following parameters:
1) quality of hair (controlled by a dermatological visit) where quality of hair means general appearance, sheen, ability to style and trend to repigmentation of the hair (thus combating whitening of the hair).
2) decrease of heterogenity of the diameters of the hairs: over the same region of the scalp some hairs have a physiological diameter and others have, in the immediate vicinity of these hairs, a decreased diameter ("fine hairs": this parameter reflects an increased tendency to hair loss).
3) increase in hair density, calculated as the number of hairs per cm²: this parameter reflects an higher regrowth of hair.
4) increase in the resistence to an applied mechanical tensile strength (pull test).
5) decrease in the loss of hair after washing with a shampoo (wash test).

### CELLULITE TREATMENT

To this end, a few notions regarding the cellulite onset should be presented. The cellulite is considered and endocrine- and metabolic- promoted alteration of the interstitial matrix. There is also a reduced efficiency of the microcirculation together with lipidema. Lipidema affects the fibroblast metabolism hence leading to perivascular proteins and glycosaminoglycans loss. This, in turn, decreases blood flow resulting in further microcirculatory slowing down and more lipogenesis that compresses the interadipocyte capillaries. The vicious circle is thus on continuous feedback. Once cellulite signs are present or even before that, a rational treatment should be established including several measures: the adoption of an adequate diet and regular moderate physical exercise practice is essential, added to localized treatment using cosmetic products.

This invention features a cosmetic treatment of the cellulite for ameliorating the unaesthetic appearances (i.e. the "orange peel" signs and the localized fat depots in certain areas of the skin), comprising daily topically applying at least one of the compositions described above onto the skin for a period of at least 30 days or more.

The compositions of the invention were found to improve the following parameters:
1) The quality of the skin (controlled by a dermatological visit), where "quality of the skin" means a decrease in both roughness and dryness and an increase in the tone of the skin.
2) Mechanical properties (skin elasticity and dermal viscoelasticity) measured by a cutomer instrument.
3) The dermal hydration state assessed by capacitance measurements with a corneometer instrument.

### AGING SKIN TREATMENT

A third aspect of the invention relates to the treatment of the unaesthetic appearances of the skin-ageing; the onset of wrinkles and age spots are two of the most visible appearances of the skin ageing.

This invention features a cosmetic treatment for ameliorating these unaesthetic appearances, comprising daily topically applying at least one of the compositions described above for a period of at least 30 days or more.

The compositions of the invention have been found to improve the following parameters:
1) Quality of the skin (controlled by a dermatological visit), where "quality of the skin" means a decrease in both roughness and dryness, an increase in the tone, a decrease in number and size of age spots, a decrease in number and size of fine lines ("wrinkles").
2) Mechanical properties (skin elasticity and dermal viscoelasticity) measured by a cutomer instrument.
3) Dermal hydration state assessed by capacitance measurements with a corneometer instrument.

## Claims

1. Cosmetic compositions for the topical use consisting of mixtures of
A) N-acyl-phosphatidylethanolamines or salts thereof, optionally mixed with phospholipids; and optionally comprising:
B) one or more bioflavones; and
C) optionally, one or more dehydrated buffering agents able to stabilize the final pH from 3.5 to 5.5.

2. Compositions as claimed in claim 1, wherein NAFE is N-oleoylphosphatidylethanolamine (NOFE) or salts thereof.

3. Compositions as claimed in claims 1-2 wherein phospholipids are selected from phosphatidylcholine, phosphatidylethanolamine, phosphatidylserine, phosphatidylinositol, phosphatidylglycerol, mixtures and corresponding lysoderivatives thereof.

4. Compositions as claimed in claims 1-3 in which bioflavones are selected from polyphenols, flavones, flavonol, isoflavones, proanthocyanidins, tannins.

5. Compositions as claimed in claims 1-4, wherein the ratio of bioflavones to phospholipid mixtures ranges from 0.5 to 20%.

6. Compositions as claimed in claims 1-4, wherein the ratio of bioflavones to phospholipid mixtures ranges from 5 to 10%.

7. Compositions as claimed in claims 1-3 and 5-6, wherein the bioflavone is epigallocatechin gallate.

8. Compositions as claimed in any one of claims 1-7, wherein bioflavones are in the form of water-dispersed phosphobioflavone complexes **characterized by** a hexagonal phase structure.

9. Compositions as claimed in any one of claims 1-7, wherein bioflavones are in the form of water-dispersed phosphobioflavone complexes **characterized by** a liposomal structure.

10. Compositions as claimed in any one of claims 1-7, wherein bioflavones are in the form of water-dispersed phosphobioflavone complexes **characterized by** a micellar structure.

11. Compositions in the form of oily emulsions containing the active principles aggregated in oily-dipsersed phosphobioflavones complexes **characterized by** a "retro-micellar" structure obtainable mixing the powders of claims 1-7 with vegetable and/or animal and/or mineral oils.

12. Compositions as claimed in claim 11, wherein the oils are selected from maize, olive, soy, bitter almond, avocado, jojoba, wheat germ, sesame, hazelnut, palm, flaxseed, vaselin, medium chain triglycerides, etc.

13. Compositions as claimed in claims 1-12, wherein the active principles, consisting of phospholipid and bioflavones mixtures and/or the complexes thereof, are further added with one or more of the following classes of compounds:
a) fibres selected from water-soluble glucosamine oligomers;
b) arginine, glutathione or mixtures thereof;
c) mixtures of mono- and di- terpenes selected from one or more of d-limonene, eugenol, thymol and phytol;
d) vitamins mixtures selected from one or more of pantotenic acid, niacinamide, biotin, tocopherols and derivatives and ascorbic acid and derivatives.

14. The use of formulations as claimed in claims 1-13 for the preparation of topical cosmetic compositions useful to improve the quality, growth and appearance of hair.

15. The use of formulations as claimed in claims 1-13 for the preparation of topical cosmetic compositions useful to prevent and/or reduce the skin unaesthetisms of cellulite.

16. The use of formulations as claimed in claims 1-13 for the preparation of topical cosmetic compositions useful to prevent and/or reduce the depth of wrinkles and the unaesthetic appearance typical of the aging skin and of the photo-aging.

17. A cosmetic method for improving the appearance of the hair and/or for promoting regrowth of the hair and/or for decreasing hair loss, comprising daily topically applying to a subject in need thereof at least one of the compositions of claims 1-13 onto the scalp and/or the hair for a period of at least 30 days or more.

18. A cosmetic method for ameliorating the unaesthetic defects caused by cellulite comprising daily topically applying to subjects in need thereof at least one of the compositions of claims 1-13 onto the skin for a period of at least 30 days or more.

19. A cosmetic method for ameliorating the unaesthetic defects caused by skin aging comprising daily topically applying to subjects in need thereof at least one of the compositions of claims 1-13 onto the skin for a period of at least 30 days or more.
